# EUROPEAN PATENT SPECIFICATION

(11) **EP 3 153 575 B1**
(45) Date of publication and mention of the grant of the patent: **24.04.2019**
(21) Application number: 15803800.0
(22) Date of filing: 03.06.2015
(51) Int. Cl.: C12N 1/21, C12P 7/40, C12N 15/09

(54) **MICROORGANISM HAVING ABILITY TO PRODUCE O-SUCCINYLHOMOSERINE OR SUCCINIC ACID, AND METHOD FOR PRODUCING SUCCINIC ACID OR O-SUCCINYLHOMOSERINE BY USING SAME**
MIKROORGANISMUS MIT FÄHIGKEIT ZUR HERSTELLUNG VON O-SUCCINYLHOMOSERIN ODER BERNSTEINSÄURE UND VERFAHREN ZUR HERSTELLUNG VON BERNSTEINSÄURE ODER O-SUCCINYLHOMOSERIN DAMIT
MICRO-ORGANISME POUVANT PRODUIRE DE LA O-SUCCINYLHOMOSÉRINE OU DE L'ACIDE SUCCINIQUE ET PROCÉDÉ DE PRODUCTION D'ACIDE SUCCINIQUE OU DE O-SUCCINYLHOMOSÉRINE AU MOYEN DE CELUI-CI

(30) Priority: 03.06.2014 KR 20140067787
(43) Date of publication of application: 12.04.2017
(73) Proprietor: CJ Cheiljedang Corporation, Seoul 100-400 (KR)
(72) Inventor: PARK, Hye Min, Gimhae-si Gyeongsangnam-do 621-805 (KR); HONG, Kuk Ki, Seoul 157-724 (KR); SONG, Gyu Hyeon, Daejeon 305-721 (KR); YANG, Young Lyeol, Goyang-si Gyeonggi-do 412-719 (KR); KANG, Min Sun, Yeosu-si Jeollanam-do 550-781 (KR); YOON, Jong Hyun, Goyang-si Gyeonggi-do 412-808 (KR); JUNG, Byung Hoon, Seoul 132-789 (KR); CHOI, Su Jin, Daegu 703-842 (KR)
(74) Representative: Potter Clarkson
(86) International application number: PCT/KR2015/005548
(87) International publication number: WO 2015/186958

(56) References cited:
- KR-A- 20060 127 989
- KR-B1- 101 136 248
- US-A- 5 977 331
- KASE H ET AL: "PRODUCTION OF O SUCCINYL-L HOMOSERINE BY AUXOTROPHIC MUTANTS OF AEROBACTER-AEROGENES", AGR. BIOL. CHEM., vol. 34, no. 2, 1970, pages 274-281, XP002767825, DOI: 10.1080/00021369.1970.10859610
- SANCHEZ SERGIO ET AL: "Metabolic regulation and overproduction of primary metabolites", MICROBIAL BIOTECHNOLOGY, vol. 1, no. 4, July 2008 (2008-07), pages 283-319, XP002767827,
- LEE SANG JUN ET AL: "Metabolic engineering of Escherichia coli for enhanced production of succinic acid, based on genome comparison and in silico gene knockout simulation", APPLIED AND ENVIRONMENTAL MICROBIOLOGY, vol. 71, no. 12, 1 December 2005 (2005-12-01), pages 7880-7887, XP002566997, AMERICAN SOCIETY FOR MICROBIOLOGY, US ISSN: 0099-2240, DOI: 10.1128/AEM.71.12.7880-7887.2005
- CHENG KE-KE ET AL: "Improved Succinate Production by Metabolic Engineering", BIOMED RESEARCH INTERNATIONAL, 2013, XP002767835,
- STEGINSKY C A ET AL: "ALPHA KETOGLUTARATE DEHYDROGENASE COMPLEX OF ESCHERICHIA-COLI A HYBRID COMPLEX CONTAINING PYRUVATE DEHYDROGENASE SUBUNITS FROM PYRUVATE DEHYDROGENASE COMPLEX", JOURNAL OF BIOLOGICAL CHEMISTRY, vol. 260, no. 25, 1985, pages 13690-13693, XP002767826, ISSN: 0021-9258
- HONG KUK-KI ET AL: "O-Succinyl-l-homoserine-based C4-chemical production: succinic acid, homoserine lactone, [gamma]-butyrolactone, [gamma]-butyrolactone derivatives, and 1,4-bu", JOURNAL OF INDUSTRIAL MICROBIOLOGY AND BIOTECHNOLOGY, vol. 41, no. 10, 26 August 2014 (2014-08-26), pages 1517-1524, XP035387180, BASINGSTOKE, GB ISSN: 1367-5435, DOI: 10.1007/S10295-014-1499-Z [retrieved on 2014-08-26]
- DATABASE NCBI 14 September 2005 Retrieved from P_709914, accession no. N N N N

## Description

### TECHNICAL FIELD

The inventive concept relates to a microorganism having an ability to produce O-succinylhomoserine or succinic acid, and a method of producing O-succinylhomoserine or succinic acid by using the same.

### BACKGROUND ART

To promote the production of O-succinylhomoserine or succinic acid under aerobic conditions, succinyl-CoA is required as a precursor. α-ketoglutarate dehydrogenase complex acts as a catalyst for the dehydrogenation of α -ketoglutarate into succinic acid in the tricarboxylic acid (TCA) cycle. The gene encoding this enzyme, sucAB, is expressed as a cluster on a chromosome along with sdhCDAB encoding succinate dehydrogenase and sucCD encoding succinyl-CoA synthetase, and this expression is known to be induced by the two promoters included in the cluster. Gene expression in the cluster is induced mainly by the sdhC promoter. At this time, sdhCDAB, sucAB, and sucCD are expressed in the stated order. The TCA cycle is advantageous for the expression of sdhCDAB, a gene encoding succinate dehydrogenase, and accordingly advantageous for the fast decomposition of succinyl-CoA because of the promoted expression of succinate dehydrogenase, even though succinyl-CoA is produced by the expression of sucAB. The sucA promoter is located between sdhCDAB and sucAB in the center of the cluster. The sucA promoter causes the expression of sucAB, but the more important promoter in this cluster is sdhC that induces the expression of sdhCDAB. The sucA promoter is weaker than the sdhC promoter (S J Park, G Chao and R P Gunsalus, J. Bacteriol. 1997; Cunningham, L. & Guest, J. R. (1998) Microbiology 144, 2113-2123). There is a report that demonstrates that the production of succinic acid was increased by the deletion of sdhAB in a succinic acid-producing strain under aerobic conditions (A. Yu. Skorofkhodova, et al., Applied Biochemistry and Microbiology, December 2013, Vol. 49, Issue 7, pp. 629-637).

In the course of research into the promotion of the production of succinic acid or O-succinylhomoserine, the present inventors developed a microorganism capable of promoting the production of O-succinylhomoserine or succinic acid by enhancing the activity of α-ketoglutarate dehydrogenase complex, leading to the completion of the present disclosure.

### DETAILED DESCRIPTION OF THE INVENTIVE CONCEPT TECHNICAL PROBLEM

It is an object of the present disclosureto provide a microorganism producing O-succinylhomoserine or succinic acid.

It is another object of the present disclosure to provide a method of producing O-succinylhomoserine or succinic acid comprising a step of culturing the microorganism having an ability to produce O-succinylhomoserine or succinic acid.

### TECHNICAL SOLUTION

According to an aspect of the inventive concept, there is provided a microorganism having an ability to produce O-succinylhomoserine or succinic acid.

In an embodiment of the present disclosure, the microorganism having an ability to produce O-succinylhomoserine or succinic acid may be a microorganism wherein an activity of α-ketoglutarate dehydrogenase complex is enhanced than an endogenous activity level thereof, thus promoting the production of O-succinylhomoserine or succinic acid.

The term "microorganism having an ability to produce O-succinylhomoserine or succinic acid" used herein indicates a prokaryotic or eukaryotic microorganism that produces O-succinylhomoserine or succinic acid in a living body and can accumulate or secrete O-succinylhomoserine or succinic acid therein. For example, the microorganism having an ability to produce O-succinylhomoserine or succinic acid may be a microorganism belonging to of the genus *Escherichia,* the genus *Erwinia,* the genus *Serratia,* the genus *Providencia,* the genus *Corynebacteria,* the genus *Pseudomonas,* the genus *Leptospira,* the genus *Salmonella,* the genus *Brevibacterium,* the genus *Hypomononas,* the genus *Chromobacterium,* the genus *Nocardia,* or fungi or yeast. In one embodiment, the microorganism according to the disclosure may be a microorganism belonging to the genus *Escherichia.* In one embodiment, the microorganism according to the disclosure may be *E. coli.*

To promote the production of succinic acid and O-succinylhomoserine, succinyl-CoA is required. Succinyl-CoA is derived by the dehydrogenation of α -ketoglutarate in the TCA cycle.

α-ketoglutarate dehydrogenase complex is a catalytic enzyme that catalyzes production of succinic acid by the dehydrogenation of α-ketoglutarate in the TCA cycle. α-ketoglutarate dehydrogenase complex is composed of α -ketoglutarate dehydrogenase and dihydrolipoic acid-succinyl group transferase, and is encoded by the sucAB (sucA GenBank Accession No. BAA35392.1, sucB GenBank Accession No. BAA35393.1) gene. α-ketoglutarate dehydrogenase and dihydrolipoic acid-succinyl group transferase may have the amino acid sequences respectively set forth in SEQ ID NO: 22 and SEQ ID NO: 24 or sequences having at least 70%, 80%, 90% or 95% homology with the sequences, which are encoded by the sucA and sucB genes. sucA and sucB may have the sequences respectively set forth in SEQ ID NO: 23 and SEQ ID NO: 25 or sequences having at least 70%, 80%, 90% or 95% homology with the sequences. sucAB is expressed as a cluster with sdhCDAB and sucCD. Succinate dehydrogenase-encoding sdhCDAB is highly expressed due to the strong promoter for sdhCDAB, and thereby succinic acid and succinyl-CoA are broken down quickly (Louise Cunningham et al., Microbiology (1998), 144, p.211-2123). Therefore, the production of succinic acid and succinyl-CoA may be promoted by increasing the production of succinic acid by enhancing the activity of the enzyme.

The term "endogenous" activity used herein indicates activity in an original microorganism or cell under natural conditions or before mutation.

In an embodiment of the present disclosure, the microorganism above is a microorganism belonging to the genus *Escherichia,* wherein an activity of homoserine O-succinyltransferase is enhanced than an endogenous activity level thereof.

Homoserine O-succinyltransferase is an enzyme acting as a catalyst for the production of O-succinylhomoserine from succinyl-CoA and homoserine, which is involved in the first step of the methionine biosynthesis pathway. The enzyme may have the amino acid sequence set forth in SEQ ID NO: 26 or an amino acid sequence having at least 70%, 80%, 90%, or 95 % homology with the sequence, and may be encoded by the metA gene (GenBank Accession No. BAE78015.1). The metA gene may have the sequence set forth in SEQ ID NO: 27 or a sequence having at least 70%, 80%, 90%, or 95% homology with the sequence. The expression of this gene is inhibited by the feedback control of methionine. Thus, a mutant engineered to increase metA expression by eliminating the feedback control of methionine has been used to promote methionine production. The methionine-mediated feedback-controlled free homoserine O-succinyltransferase may have the amino acid sequence set forth in SEQ ID NO: 32 or a sequence having at least 70%, 80%, 90%, or 95% homology with the sequence, and its gene may be encoded by the sequence set forth in SEQ ID NO: 33 (metA11: Korean Patent Publication NO. 2009-0106365) or a sequence having at least 80%, 90%, or 95% homology with the sequence.

The term "homology" used in relation to a sequence used herein indicates the degree of matching with the given amino acid sequence or nucleotide sequence, which may be presented as a percentage (%). In this disclosure, a homology sequence having an activity which is identical or similar to the given amino acid sequence or nucleotide sequence is presented as "% homology", which may be easily determined by those in the art capable of using the BLAST 2.0 computer program that calculates parameters such as score, identity, and similarity.

The terms "enhance" or "strengthen" for enzyme activity used herein indicates an increase in enzyme activity to a level higher than that of the endogenous activity, which may result from overexpression of a gene encoding an enzyme or an increase in the enzyme activity to a level higher than the endogenous activity level by gene mutation. For example, the number of copies of the gene encoding an enzyme may be increased, or the endogenous promoter of the gene may be replaced with a stronger promoter; alternatively, a whole sequence or a part of the sequence of the gene or a whole expression regulatory sequence or a part of it on the chromosome may be mutated by deletion, substitution, or insertion, or by a combination thereof.

The term "expression regulatory sequence" used herein indicates a nucleotide sequence regulating gene expression, which is a segment that can increase or decrease the expression of a specific gene in an organism, and includes a promoter, a transcription factor binding site, etc, but not limited thereto.

In an embodiment of the present disclosure, a promoter of a gene encoding a corresponding enzyme may be replaced with a stronger promoter than the endogenous promoter or the number of copies of the gene may be increased in order to increase the corresponding enzyme activity.

In an embodiment of the present disclosure, the promoter may be Ptac, Ptrc, Ppro, PR, PL, Prmf, or PcysK, which are all known as promoters with strong activity, but not limited thereto.

In an embodiment of the present disclosure, the microorganism may be a microorganism belonging to the genus *Escherichia,* wherein activity of one or more enzymes of cystathionine gamma-synthase and homoserine kinase is weakened, as compared with corresponding endogenous activity level, or eliminated.

Cystathionine gamma-synthase catalyzes the conversion of O-succinylhomoserine to cystathionine. Cystathionine gamma-synthase may have the amino acid sequence set forth in SEQ ID NO: 28 or a sequence having at least 70%, 80%, 90%, or 95% homology with the sequence, and may be encoded by the metB gene (GenBank Accession No. BAE77371.1). The metB gene may have the sequence set forth in SEQ ID NO: 29 or a sequence having at least 70%, 80%, 90%, or 95% homology with the sequence. Once the activity of this enzyme is weakened or eliminated, O-succinylhomoserine is not converted into cystathionine or, if converted, the conversion rate is very low.

Homoserine kinase acts as a catalyst for the synthesis of O-phosphohomoserine from homoserine, and may have the amino acid sequence set forth in SEQ ID NO: 30 or a sequence having at least 70%, 80%, 90%, or 95% homology with the sequence and which may be encoded by thrB gene (GenBank Accession No. BAB96580.1). The thrB gene may have the sequence set forth in SEQ ID NO: 31 or a sequence having at least 70%, 80%, 90%, or 95% homology with the sequence. Once the activity of this enzyme is weakened or eliminated, homoserine is not converted into O-phosphohomoserine or, if converted, the conversion rate is very low.

The terms "weakened" or "eliminated" of enzyme activity used herein indicate a decrease of the expression of a gene encoding its corresponding enzyme to a level lower than the endogenous level or a complete removal of the enzyme activity, respectively. This may be achieved by mutating a whole nucleotide sequence or a part of a nucleotide sequence, or a whole or a part of an expression regulatory sequence by deleting, replacing, inserting, or by a combination thereof.

In an embodiment of the present disclosure, the microorganism may be *E. coli.* The microorganism may be engineered to increase the activity of α-ketoglutarate dehydrogenase complex to a level higher than the endogenous level and additionally to enhance the activity of homoserine O-succinyltransferase stronger to a level greater than the endogenous level; or the activity of one or both enzymes of cystathionine gamma-synthase and homoserine kinase may be weakened to a level lower than the endogenous level or eliminated.

In an embodiment of the present disclosure, the microorganism of the disclosure may have the metA gene on the chromosome of E. coli replaced with the mutant metA11 (SEQ ID NO: 33, WO2008-127240 A1) for which feedback regulation mediated by methionine has been eliminated, may have thrB and metB genes deleted from the chromosome, and may be transformed with a vector containing sucAB that is under control of a promoter being stronger than its original promoter.

An aspect of the present disclosure provides a method of producing O-succinylhomoserine or succinic acid, the method including culturing a microorganism of the genus *Escherichia* in a media, wherein an activity of α-ketoglutarate dehydrogenase complex is enhanced as compared with an endogenous activity level thereof, and recovering O-succinylhomoserine or succinic acid from the culture media or the cultured microorganism.

In an embodiment of the present disclosure, in the method for producing O-succinylhomoserine or succinic acid, the culturing of a microorganism having an ability to produce O-succinylhomoserine or succinic acid may be achieved by using a proper medium and culture conditions well known to those in the art. This culture process may be easily regulated according to the strain selected by those in the art. As examples of the culture method, batch culture, continuous culture, and fed-batch culture are all included, but not limited thereto. Various culture methods including the above are explained in a reference ("Biochemical Engineering" by James M. Lee, Prentice-Hall International Editions, pp 138-176.).

The culture medium herein has to satisfy the culture condition required for the culture of a specific strain herein. Various culture media for microorganisms are described in a reference ("Manual of Methods for General Bacteriology" by the American Society for Bacteriology, Washington D.C., USA, 1981.). These media include various carbon sources, nitrogen sources, and trace elements. The carbon source includes carbohydrates such as glucose, lactose, sucrose, fructose, maltose, starch, and cellulose; fats such as soybean oil, sunflower oil, castor oil, and coconut oil; fatty acids such as palmitic acid, stearic acid, and linoleic acid; alcohols such as glycerol and ethanol; and organic acids such as acetic acid. The carbon source may be used independently or as a combination of at least two carbon sources. The nitrogen source includes organic nitrogen sources such as peptone, yeast extract, gravy, malt extract, corn steep liquor (CSL), and bean flour; and inorganic nitrogen sources such as urea, ammonium sulfate, ammonium chloride, ammonium phosphate, ammonium carbonate, and ammonium nitrate. These nitrogen sources may be used alone or in combination. In addition, a phosphorus source may be additionally included in the culture medium above, and examples thereof include potassium dihydrogen phosphate, dipotassium hydrogen phosphate, and the corresponding sodium-containing salts. The culture medium herein can also contain a metal such as magnesium sulfate or iron sulfate. Additionally, the culture medium can include amino acids, vitamins, and proper precursors.

To maintain the aerobic condition in the culture fluid, oxygen or oxygen-containing gas (air) may be injected into the culture medium. The temperature for the culture herein is generally 20 to 45°C and preferably 25 to 40 °C. The culturing may be continued until production of O-succinylhomoserine or succinic acid reaches a desired level, and preferred culturing time is 10 hrs to 160 hrs.

### ADVANTAGEOUS EFFECTS

The O-succinylhomoserine- or succinic acid-producing strain according to the present disclosure is efficient in producing O-succinylhomoserine or succinic acid and may be applied in various fields due to possibilities for enzymatic or chemical conversion.

### DESCRIPTION OF THE DRAWINGS

FIG. 1 is a schematic diagram of the recombinant vector pCL_PsucA-sucAB.
FIG. 2 is a schematic diagram of the recombinant vector pCL_Prmf-sucAB.
FIG. 3 is a schematic diagram of the recombinant vector pCL_Ptrc-sucAB.
FIG. 4 is a schematic diagram of the recombinant vector pCL_Pcysk-metA11_PsucA-sucAB.

### MODE OF THE INVENTIVE CONCEPT

Hereinafter, the present disclosure will be described in more detail with reference to Examples. However, these Examples are for illustrative purposes only, and the scope of the present disclosure is not intended to be limited by these Examples.

### Reference Example. Construction of O-succinylhomoserine- or succinic acid-producing strain

### 1) Deletion of metB gene

To increase the accumulation of O-succinylhomoserine or succinic acid, a strain was constructed by deleting metB gene encoding cystathionine gamma-synthase involved in the degradation of L-methionine precursor.

The metB gene encoding cystathionine gamma-synthase in the wild-type *E. coli* (K12) W3110 strain was deleted. It is known that Cystathionine gamma-synthase binds to various methionine precursors and thereby can produce various by-products. Thus, the overexpression of cystathionine synthase might increase side reactions to reduce efficiency of intracellular reactions. To delete the metB gene, FRT-one-step-PCR deletion method was performed (PNAS (2000), Vol. I97, p6640-6645). To delete the metB gene, PCR was performed with the primers represented by SEQ ID NO: 12 and SEQ ID NO: 13 using the pKD3 vector (PNAS (2000) Vol. 97, p6640-6645) as a template, resulting in the construction of a deletion cassette.

PCR was performed as follows; denaturation at 95°C for 30 seconds, annealing at 55°C for 30 seconds, and extension at 72°C for 1 minute, with 30 cycles performed from denaturation to extension. The PCR product underwent electrophoresis on 1.0% agarose gel. The 1.1 kb band was eluted and purified. The recovered DNA fragment was electroporated into the *E. coli* (K12) W3110 strain previously transformed with the pKD46 vector (PNAS (2000) Vol. 97, p6640-6645). For the electroporation, the W3110 strain transformed with pKD46 was cultured in LB medium supplemented with 200 *µ*g/L ampicillin and 5 mM L-arabinose at 30°C until OD₆₀₀ reached 0.5. The strain was washed with 10% glycerol three times. Electroporation was performed at 2500 V. The recovered strain was spread on LB plate medium containing 30 *µ*g/L chloramphenicol, foll*owed by* culturing at 37°C for 1 to 2 days. Then, the resistant strain was selected.

The selected strain was then used for PCR using the primers represented by SEQ ID NO: 14 and SEQ ID NO: 15 according to the conditions mentioned above.
A band of a size of 1.5 kb was observed on 1.0% agarose gel, suggesting that the metB gene was deleted.
SEQ ID NO: 14; 5'-TATTCGCCGCTCCATTCAGC-3'
SEQ ID NO: 15; 5'-TACCCCTTGTTTGCAGCCCG-3'

The metB gene-deleted strain was transformed with the pCP20 vector (PNAS (2000) vol.97, p6640-6645), which was then cultured in LB medium supplemented with 100 *µ*g/L ampicillin. PCR was performed by the same manner as described above and the final metB gene-deleted strain, exhibiting a shrunk band on 1.0% agarose gel, was selected. The deletion of chloramphenicol marker was confirmed. The obtained methionine-auxotrophic strain was named CC03-0132.

### 2) Deletion of thrB gene

To increase the production of O-succinyl homoserine from homoserine, thrB, the gene encoding homoserine kinase, was deleted. In particular, when a threonine-producing strain is used, the deletion of this gene is necessary because homoserine utilization activity is very strong. To delete the thrB gene from the CC03-0132 strain constructed in 1) above, FRT-one-step-PCR deletion method (PNAS (2000) Vol. 97, p6640-6645) was performed. To delete the thrB gene, PCR was performed with the primers represented by SEQ ID NO: 16 and SEQ ID NO: 17 using the pKD3 vector (PNAS (2000) Vol. 97, p6640-6645) as a template, resulting in the construction of a deletion cassette.

PCR was performed as follows; denaturation at 95°C for 30 seconds, annealing at 55°C for 30 seconds, and extension at 72°C for 1 minute, with 30 cycles performed from denaturation to extension. The PCR product underwent electrophoresis on 1.0% agarose gel. The 1.1 kb band was eluted and purified. The recovered DNA fragment was electroporated into the CC03-0132 strain previously transformed with the pKD46 vector (PNAS (2000) Vol. 97, p6640-6645). For the electroporation, the CC03-0132 strain transformed with pKD46 was cultured in LB medium supplemented with 200 *µ*g/L ampicillin and 5 mM L-arabinose at 30°C until OD₆₀₀ reached 0.5. The strain was washed with 10% glycerol three times. Electroporation was performed at 2500 V. The recovered strain was spread on LB plate medium containing 30 *µ*g/L chloramphenicol, followed by culturing at 37°C for 1 to 2 days. Then, the resistant strain was selected.

The selected strain proceeded to PCR using the primers represented by SEQ ID NO: 18 and SEQ ID NO: 19 according to the conditions mentioned above. A band of a size of 1.5 kb was observed on 1.0% agarose gel, suggesting that the thrB gene was deleted.
SEQ ID NO: 18; 5'-ACTCGACGATCTCTTTGCC-3'
SEQ ID NO: 19; 5'-ACGCCGAGAGGATCTTCGCAG-3'

The confirmed strain was transformed with the pCP20 vector (PNAS (2000) vol. 97, p6640-6645), which was then cultured in LB medium supplemented with 100 *µ*g/L ampicillin. PCR was performed thereon in the same manner as described above and the final thrB gene-deleted strain, exhibiting a smaller band as confirmed on 1.0% agarose gel electrophoresis, was selected. The deletion of the chloramphenicol marker was confirmed. The obtained strain was named CC03-0133.

### 3) Construction of pSG vector for inserting metA11

Homoserine succinyl transferase is under feedback control by trace methionine added to the medium so that most of the activity of homoserine succinyl transferase is inhibited. To increase the production of O-succinyl homoserine, the L-methionine precursor, a mutant free from feedback control by methionine, was used. To replace the wild-type metA gene (SEQ ID NO: 27) on the chromosome encoding homoserine succinyl transferase in E. coli with the metA11 (SEQ ID NO: 33) mutant which is free from feedback control by methionine, the insertion vector pSG-metA11 was constructed. According to the instruction given in WO2008/127240 A1, the nucleotide sequence information of the metA11 gene was obtained, and based on the information, the primers (SEQ ID NO: 20 and SEQ. ID NO: 21) were synthesized containing the open reading frame (ORF) starting from the start codon ATG of the metA11 gene and the recognition sites of restriction enzymes, EcoRI and Sacl. PCR was performed with the primers represented by the following sequences using TF4076BJF metA#11 strain shown in WO2008/127240 A1 as a template.
SEQ ID NO: 20; 5'-ggccgaattcatgccgattcgtgtgccgga-3'
SEQ ID NO: 21; 5'-ggccgagttcttaatccagcgttggattca-3'

PCR was performed using pfu-X DNA polymerase (Solgent, SPX16-R250) as follows: denaturation at 95°C for 30 seconds, annealing at 55°C for 30 seconds, and extension at 72°C for 2 minutes, with 30 cycles from denaturation to extension. As a result, the PCR product in which the metA11 ORF containing the recognition sites of EcoRI and Sacl at both ends was amplified, was obtained. The metA11 gene obtained by PCR above was treated with the restriction enzymes EcoRI and Sacl, followed by ligation into the pSG76-C vector (J Bacteriol. 1997 Jul;179(13):4426-8.) which had also been treated with the restriction enzymes EcoRI and Sacl. Finally, the pSG-metA11 recombinant vector containing the cloned metA11 gene was constructed.

### 4) Construction of metA11 insertion strain

The metA11 insertion vector pSG-metA11 constructed in Reference Example 3) was introduced into the strain obtained in Reference Example 2), followed by culture in LB-Cm medium (yeast extract 10 g/L, NaCl 5 g/L, tryptone 10 g/L, chloramphenicol 30 *µ*g/L). Then, chloramphenicol-resistant colonies were selected. The selected transformant was the primary strain inserted with the pSG-metA11 vector in the position of metA of the chromosome. The strain introduced with the metA11 gene was transformed with the pASceP vector (JOURNAL OF BACTERIOLOGY, July 1997, 4426-4428) expressing the restriction enzyme I-Scel to cleave the I-Scel site in the pSG vector. The strain was selected after being grown in LB-Amp medium (yeast extract 10 g/L, NaCl 5 g/L, tryptone 10 g/L, chloramphenicol 100 *µ*g/L). In the selected strain, the wild-type metA was replaced with metA11 and the inserted pSG76-C vector was eliminated therefrom. This strain was named E. coli CC03-0038.

### 5) Construction of O-succinylhomoserine-producing strain based on threonine-producing strain

A strain having an ability to produce succinic acid and O-succinyl homoserine was constructed by the same method as described in 1) to 4) above by using E. coli KCCM 10541P, the threonine production strain, described in International Patent WO 2005/075625, instead of the wild-type strain W3110, and the constructed strain was named CJM2-A11.

### Example 1. Construction of a plasmid for the enhanced expression of sucAB

### 1-1. Construction of a plasmid for the enhanced expression of sucAB with a sucA promoter

The nucleotide sequence information of α-ketoglutarate dehydrogenase complex encoded by sucAB (sucA GenBank Accession No. BAA35392.1: SEQ ID NO: 23, sucB GenBank Accession No. BAA35393.1: SEQ ID NO: 25) was obtained from the database of the National Center for Biotechnology Information, USA, based on which the primers, represented by SEQ ID NO: 1 and SEQ ID NO: 2, and recognized by the restriction enzymes Hindlll and Xbal respectively and containing the sequence ranging -188 from ATG, the ORF initiation codon of sucAB, were synthesized in order to obtain the sucAB gene under the control of the sucA promoter.
SEQ ID NO: 1; 5'-GGCCAAGCTTGCATCAGGCGTAACAAAGAA-3'
SEQ ID NO: 2; 5'-GGCCTCTAGATGTCCATCCTTCAGTAATCG-3'

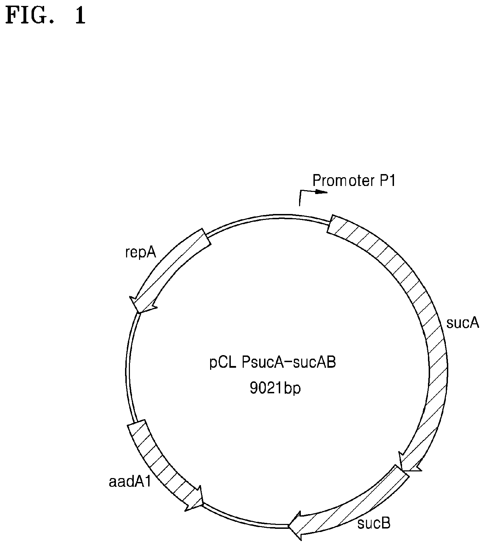

Using the chromosomal DNA of wild-type E. coli W3110 as a template, the cloning of sucAB, the gene encoding α-ketoglutarate dehydrogenase complex, was performed by PCR with the primers represented by SEQ ID NO: 1 and SEQ ID NO: 2. PCR [Sambrook et al, Molecular Cloning, a Laboratory Manual (1989), Cold Spring Harbor Laboratories] was performed using pfu-X DNA polymerase (Solgent, SPX16-R250) as follows: denaturation at 95°C for 30 seconds, annealing at 55°C for 30 seconds, and extension at 72°C for 5 minutes, with 30 cycles from denaturation to extension. As a result, an approximately 4.5 kb PCR product containing the sucA promoter, the sucAB gene, and the recognition sites of Hindlll and Xbal was obtained. The obtained PCR product was treated with the restriction enzymes Hindlll and Xbal. By using T4 DNA ligase, the PCR product was ligated into the pCL1920 vector (Lerner, C.G. and Inouye, M., Nucl. Acids Res.(1990) 18:4631) which had been pre-treated with the restriction enzymes Hindlll and Xbal, resulting in the construction of the recombinant vector pCL PsucA-sucAB. FIG. 1 is a schematic diagram illustrating the recombinant vector pCL PsucA-sucAB.

### 1-2. Construction of a plasmid for the enhanced expression of sucAB with a promoter rmf or trc

The nucleotide sequence information (sucA GenBank Accession No. BAA35392.1: SEQ ID NO: 23, sucB GenBank Accession No. BAA35393.1: SEQ ID NO: 25) of the sucAB gene (the gene encoding α-ketoglutarate dehydrogenase complex) was obtained from the database of the National Center for Biotechnology Information, USA, based on which the primers represented by SEQ ID NO: 3 and SEQ ID NO: 4 having the recognition sites of EcoRV and HindIII were synthesized in order to obtain the sucAB gene.
SEQ ID NO: 3; 5'-ATCATGCAGAACAGCGCTTTGAA-3'
SEQ ID NO: 4; 5'-GGCCAAGCTTTGTCCATCCTTCAGTAATCG-3'

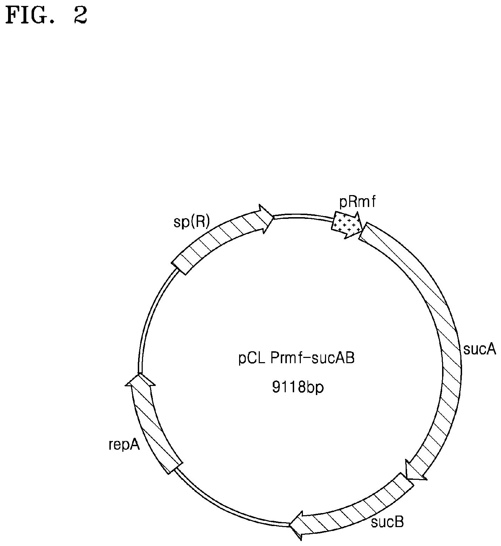

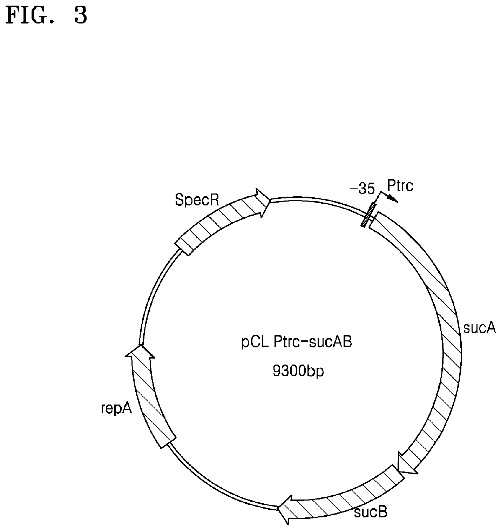

Using the chromosomal DNA of wild-type E. coli W3110 as a template, the cloning of sucAB was performed by PCR with the primers represented by SEQ ID NO: 3 and SEQ ID NO: 4 . PCR [Sambrook et al, Molecular Cloning, a Laboratory Manual (1989), Cold Spring Harbor Laboratories] was performed using pfu-X DNA polymerase (Solgent, SPX16-R250) as follows: denaturation at 95°C for 30 seconds, annealing at 55°C for 30 seconds, and extension at 72°C for 5 minutes, with 30 cycles from denaturation to extension. As a result, an approximately 4.3 kb PCR product containing the sucAB gene and the recognition site of restriction enzyme HindIII was obtained. The obtained PCR product was treated with the restriction enzyme HindIII. To replace the sucA promoter, that is, the native promoter of the sucAB gene, the vectors pCL_Prmf-gfp (SEQ ID NO: 5) and pCL_Ptrc-gfp (SEQ ID NO: 6) containing respectively promoters rmf and trc were treated with the restriction enzymes EcoRV and Hindlll, followed by ligation of the PCR product using T4 DNA ligase (Roche: 10481220001). As a result, the recombinant vectors pCL_Prmf-sucAB and pCL_Ptrc-sucAB were constructed. The vectors pCL_Prmf-gfp and pCL_Ptrc-gfp were introduced with the green fluorescence protein gene gfp in order to measure the strength of the promoters rmf and trc. In the meantime, sucAB was ligated with the promoter region of the vector, resulting in the construction of a vector containing sucAB that would be expressed under the control of the promoters rmf and trc. FIG. 2 and FIG. 3 are schematic diagrams illustrating the recombinant vectors pCL_Prmf-sucAB and pCL_Ptrc-sucAB, respectively.

### Example 2. Construction of a plasmid for the simultaneously enhanced expression of sucAB and metA11

To synthesize O-succinylhomoserine, an expression vector expressing sucAB and metA11 simultaneously was constructed. The nucleotide sequence information of the metA11 gene was obtained based on the amino acid sequence encoding the O-succinyl transferase mutant of the TF4076BJF metA#11 strain described in International Patent Publication No. WO2008/127240 A1, and, based on the nucleotide sequence information, the primers represented by SEQ ID NO: 7 and SEQ ID NO: 8 having the recognition sites of restriction enzymes EcoRV and HindIII at both ends were synthesized in order to amplify the ORF ranging from ATG to TAA of the metA11 gene.
SEQ ID NO: 7; 5'-GAGTGCGATATC atgccgattcgtgtgccggac-3'
SEQ ID NO: 8; 5'-GCACTCAAGCTT ttaatccagcgttggatacatg-3'

Using TF4076BJF metA#11 as a template, PCR was performed with the primers represented by SEQ ID NO: 7 and SEQ ID NO: 8. PCR was performed using pfu-X DNA polymerase (Solgent, SPX16-R250) as follows: denaturation at 95°C for 30 seconds, annealing at 55°C for 30 seconds, and extension at 72°C for 1 minute, with 30 cycles from denaturation to extension. The obtained PCR product was treated with the restriction enzymes EcoRV and Hindlll. The vector pCL_Pcysk-gfp (SEQ ID NO: 9) was treated with the restriction enzymes EcoRV and Hindlll, followed by ligation using T4 DNA ligase (Roche: 10481220001). As a result, the recombinant vector pCL_Pcysk-metA11 was constructed. To insert sucAB in the constructed vector above, the primers represented by SEQ ID NO: 10 and SEQ ID NO: 11 having the recognition site of HindIII were synthesized.
SEQ ID NO: 10; 5'-GGCCAAGCTTGCATCAGGCGTAACAAAGAA-3'
SEQ ID NO: 11; 5'-GGCCAAGCTTTGTCCATCCTTCAGTAATCG-3'

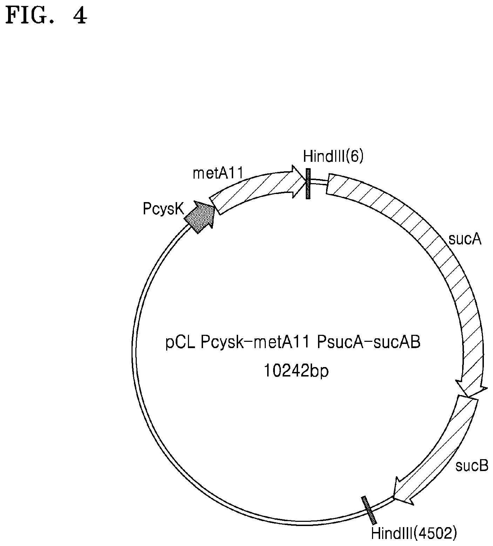

To express metA11 and sucAB simultaneously, the vector pCL_Pcysk-metA11_PsucA-sucAB was constructed. First, using the chromosomal DNA of wild-type E. coli W3110 as a template, PCR was performed with the primers represented by SEQ ID NO: 10 and SEQ ID NO: 11. PCR [Sambrook et al, Molecular Cloning, a Laboratory Manual (1989), Cold Spring Harbor Laboratories] was performed using pfu-X DNA polymerase (Solgent, SPX16-R250) as follows: denaturation at 95°C for 30 seconds, annealing at 55°C for 30 seconds, and extension at 72°C for 5 minutes, with 30 cycles from denaturation to extension. As a result, a PCR product of approximately 4.5 kb having PsucA-sucAB including the recognition site of HindIII was obtained. The obtained PCR product was treated with the restriction enzyme HindIII, followed by ligation, using T4 DNA ligase (Roche:10481220001), into the pCL_Pcysk-metA11 vector which had been pre-treated with the restriction enzyme HindIII. As a result, the recombinant vector pCL_Pcysk-metA11_PsucA-sucAB was constructed. FIG. 4 is a schematic diagram illustrating the recombinant vector pCL_Pcysk-metA11_PsucA-sucAB.

### Example 3. Fermentation for the production of succinic acid

Flask culture was performed to investigate the production of succinic acid when activity of only sucAB was enhanced in the O-succinylhomoserine-producing strain constructed in the Reference Example. The strain of Reference Example 4), CC03-0038, and the strain of Reference Example 5), CJM2-A11, were transformed with the plasmids constructed in Example 1, pCL_PsucA-sucAB, pCL_Prmf-sucAB, and pCL_Ptrc-sucAB. The strains were spread on an LB plate medium containing spectinomycin, resulting in strains inserted with sucAB. As controls, prepared were CC03-0038 and CJM2-A11 strains introduced with the pCL1920 vector. The pCC1BAC-scrO vector (SEQ ID NO: 34) having the scrO sequence of the pUR 400 plasmid originating from the salmonella strain described in International Patent Publication No. WO10/101360 was introduced into these strains to make them to use raw sugar as a carbon source. The resultant strains were cultured in the medium having the composition shown in Table 2 below for the evaluation of the production of succinic acid.

Each strain was inoculated into the medium and cultured at 33°C overnight. A single colony was inoculated into 2 ml of LB medium containing spectinomycin, followed by culture at 33°C for 2 hours. The strain was inoculated again into a 250 ml Erlenmeyer flask containing 25 ml flask medium at a density of OD₆₀₀=0.5, followed by culture at 33°C at 200 rpm for 33 hours. High Performance Liquid Chromatography (HPLC) was performed to investigate the production of succinic acid. The results are shown in Table 3.

As a result, as expression of sucAB was enhanced, the production of succinic acid was increased. This result indicates that the enhanced expression of sucAB has the effect of down-regulating glutamate but increasing the flux of succinyl-CoA production to increase the production of succinic acid. When glucose was used as a carbon source, the production of succinic acid increased by 30% at maximum. The production of succinic acid was expected to be further increased as succinic acid synthesis was enhanced in a strain under aerobic conditions. When raw sugar was used as a carbon source, the production of succinic acid increased as much as when glucose was used as a carbon source. The results are shown in Table 4.

### Table 1. Composition of glucose flask medium

**[Table 1]**

| Composition | Stock | Cone (per liter) | Vol (ml) |
|---|---|---|---|
| Glucose | | 40 g | 200 |
| KH₂PO₄ | | 2 g | 100 |
| Ammonium sulfate | | 17 g | 500 |
| MgSO₄·7H₂O | | 1 g | |
| Yeast extract | | 2 g | |
| Methionine | | 0.4 g | |
| Threonine | | 1 g | |
| MnSO₄·7H₂O | 10 mg/ml | 0.01 g (stock 1 ml) | |
| ZnSO₄·7H₂O | 1 mg/ml | 0.01 g (stock 10 ml) | |
| FeSO₄·7H₂O | 10 mg/ml | 10 mg (stock 1 ml) | |
| Calcium carbonate | | 30 g | 200 |

### Table 2. Composition of raw sugar flask medium

**[Table 2]**

| Composition | Stock | Cone (per liter) | Vol (ml) |
|---|---|---|---|
| Raw sugar | | 60g | 200 |
| KH₂PO₄ | | 2 g | 100 |
| Ammonium sulfate | | 25g | 500 |
| MgSO₄·7H₂O | | 1 g | |
| Yeast extract | | 2 g | |
| methionine | | 0.4 g | |
| threonine | | 1 g | |
| MnSO₄·7H₂O | 10 mg/ml | 0.01 g (stock 1 ml) | |
| ZnSO₄·7H₂O | 1 mg/ml | 0.01 g (stock 10 ml) | |
| FeSO₄·7H₂O | 10 mg/ml | 10 mg (stock 1 ml) | |
| Calcium carbonate | | 30 g | 200 |

### Table 3. Succinic acid production via flask culture using glucose

**[Table 3]**

| Strain | Glucose (g/L) | O-succinic acid (g/L) | O-succinyl homoserine (g/L) | Glutamate (g/L) |
|---|---|---|---|---|
| CC03-0038/pCL1920 | 40 | 0.30 | 4.5 | 1.87 |
| CC03-0038/pCL_ PsucA-sucAB | 40 | 0.63 | 4.9 | 0.0 |
| CC03-0038/pCL-Prmf-sucAB | 40 | 0.88 | 5.2 | 0.0 |
| CC03-0038/pCL_Ptrc-sucAB | 40 | 0.93 | 5.5 | 0.0 |
| CJM2-A11/pCL1920 | 40 | 0.012 | 10.9 | 0.84 |
| CJM2-A11/pCL_PsucA-sucAB | 40 | 0.26 | 11.5 | 0.0 |
| CJM2-A11/pCL-Prmf-sucAB | 40 | 0.63 | 12.1 | 0.0 |
| CJM2-A11/pCL_Ptrc-sucAB | 40 | 0.77 | 12.5 | 0.0 |

### Table 4. Succinic acid production via flask culture using raw sugar

**[Table 4]**

| Strain | Raw sugar (g/L) | O-succinic acid(g/L) | O-succinyl homoserine (g/L) | Glutamate (g/L) |
|---|---|---|---|---|
| CC03-0038/pCC1BAC-scrO/pCL1920 | 60 | 0.24 | 7.0 | 1.33 |
| CC03-0038/pCC1 BAC-scrO/pCL_ PsucA-sucAB | 60 | 0.312 | 7.5 | 0 |
| CC03-0038/pCC1 BAC-scrO/pCL_ Prmf-sucAB | 60 | 0.762 | 7.9 | 0 |
| CC03-0038/pCC1 BAC-scrO/pCL_ Ptrc-sucAB | 60 | 1.56 | 8.2 | 0 |
| CJM2-A11/pCC1BAC-scrO/pCL1920 | 60 | 0.023 | 15.7 | 0.77 |
| CJM2-A11/pCC1BAC-scrO/pCL_PsucA-sucAB | 60 | 0.22 | 16.1 | 0 |
| CJM2-A11/pCC1BAC-scrO/pCL-Prmf-sucAB | 60 | 0.53 | 16.7 | 0 |
| CJM2-A11/pCC1BAC-scrO/pCL_Ptrc-sucAB | 60 | 1.07 | 17.0 | 0 |

### Example 4. Fermentation for the production of O-succinyl homoserine

To enhance the expression of sucAB and metA genes together in the CC03-0038 and CJM2-A11 strains, the strains were transformed with the pCL_Pcysk-metA11_PsucA-sucAB plasmid constructed in Example 2, followed by culturing in an Erlenmeyer flask to investigate the production of O-succinyl homoserine. The composition of the flask medium was as shown in Table 1. The enhanced expression of metA11 gene in Reference Example 4) was under the control of the original promoter. Therefore, in order to enhance its expression, metA11 was cloned into a vector where it could be expressed by the cysK promoter, wherein the expression of sucAB was simultaneously induced.

CC03-0038 was transformed with the pCL_Pcysk-metA11_PsucA-sucAB plasmid constructed in Example 2. The strain was spread on the LB plate medium containing spectinomycin, resulting in the preparation of the transformed strain. As for the controls, CC03-0038 and CJM2-A11 were transformed with the pCL1920 vector. A strain that could use raw sugar as a carbon source was constructed by introducing the pCC1BAC-scrO vector having the scrO sequence of the pUR 400 plasmid originating from the salmonella strain described in Korean Patent Application No. 2009-0018128 into the strains. The strain was cultured in the raw sugar flask medium having the composition shown in Table 2 for the evaluation of the production of O-succinyl homoserine. For evaluation purpose, some of the strains described above were inoculated into the medium and cultured at 33°C overnight. A single colony was inoculated into 2 ml of LB medium containing spectinomycin, followed by culture at 33°C for 2 hours. The strains were inoculated again into a 250 ml Erlenmeyer flask containing 25 ml flask medium at a density of OD₆₀₀=0.5, followed by culture at 33°C at 200 rpm for 33 hours. HPLC was performed to investigate the production of O-succinylhomoserine. The results are shown in Table 5.

The results indicated that, in a strain introduced with pCL_Pcysk-metA11_PsucA-sucAB, O-succinylhomoserine was up-regulated. The production yield was increased by about 40% as compared with the control. As O-succinylhomoserine was increased, glutamate and homoserine were down-regulated. The results indicate that the enhanced expression of sucAB plays an important role in supplying succinyl-CoA. When expression of metA, using succinyl-CoA as a substrate, was simultaneously enhanced, the concentration of O-succinylhomoserine rapidly increased.

### Table 5. O-succinylhomoserine production via flask culture using glucose

**[Table 5]**

| Strain | O-succinyl homoserine (g/L) | Glutamate (g/L) | Homoserine (g/L) |
|---|---|---|---|
| CC03-0038/pCL1920 | 4.5 | 1.87 | 0.4 |
| CC03-0038/pCL_Pcysk-metA11_PsucA-sucAB | 6.31 | 0.0 | 0.17 |
| CJM2-A11/pCL1920 | 10.9 | 0.84 | 1.1 |
| CJM2-A11/pCL_Pcysk-metA11_PsucA-sucAB | 15.8 | 0.0 | 0.5 |

### Table 6. O-succinylhomoserine production via flask culture using raw sugar

**[Table 6]**

| Strain | O-succinyl homoserine (g/L) | Glutamate (g/L) | Homoserine(g/L) |
|---|---|---|---|
| CC03-0038/pCC1 BAC-scrO/pCL1920 | 7.0 | 1.87 | 0.7 |
| CC03-0038/pCC1 BAC-scrO/pCL_Pcysk-metal11_PsucA-sucAB | 12.4 | 0.0 | 0.23 |
| CJM2-A11/pCC1BAC-scrO/pCL1920 | 15.7 | 0.84 | 1.5 |
| CJM2-A11/pCC1BAC-scrO/pCL_Pcysk-metA11_Pna-sucAB | 22.6 | 0.0 | 0.54 |

When a microorganism belonging to the genus *Escherichia* was transformed with a vector containing sucAB according to an embodiment of the present disclosure to enhance the expression of sucAB, the production of succinyl-CoA and succinic acid was increased. Therefore, the enhanced expression of sucAB may be applied to those microorganisms that have the same TCA cycle in regard to central carbon metabolism as the above, for example to those microorganisms such as yeast and Actinomyces, etc.

The engineered CC03-0038/pCL_Pcysk-metA11_PsucA-sucAB strain, confirmed to be capable of producing O-succinyl homoserine, was named CC03-0157, and was deposited at the Korean Culture Center of Microorganisms (KCCM) under the Budapest Treaty on November 22, 2013 (Accession No: KCCM11488P).

### Sequence Listing Free Text

The sequences represented by SEQ ID NO: 1 to SEQ ID NO: 34 described used herein are shown in the attached sequence list.
<110> CJ CHEILJEDANG Corporation
<120> Microorganism capable of producing O-succinylhomoserine or succinic acid and method of producing O-succinylhomoserine or succinic acid
<130> PN103856
<160> 34
<170> KopatentIn 2.0
<210> 1
   <211> 30
   <212> DNA
   <213> Artificial Sequence
<220>
   <223> Forward primer for Psuc_sucAB
<400> 1
   ggccaagctt gcatcaggcg taacaaagaa 30
<210> 2
   <211> 30
   <212> DNA
   <213> Artificial Sequence
<220>
   <223> Reverse primer for Psuc_sucAB
<400> 2
   ggcctctaga tgtccatcct tcagtaatcg 30
<210> 3
   <211> 23
   <212> DNA
   <213> Artificial Sequence
<220>
   <223> Forward primer for sucAB
<400> 3
   atcatgcaga acagcgcttt gaa 23
<210> 4
   <211> 30
   <212> DNA
   <213> Artificial Sequence
<220>
   <223> Reverse primer for sucAB
<400> 4
   ggccaagctt tgtccatcct tcagtaatcg 30
<210> 5
   <211> 5545
   <212> DNA
   <213> Artificial Sequence
<220>
   <223> pCL_Prmf-gfp
<210> 6
   <211> 5727
   <212> DNA
   <213> Artificial Sequence
<220>
   <223> pCL_Ptrc-gfp
<210> 7
   <211> 33
   <212> DNA
   <213> Artificial Sequence
<220>
   <223> Forward primer for metA11
<400> 7
   gagtgcgata tcatgccgat tcgtgtgccg gac 33
<210> 8
   <211> 34
   <212> DNA
   <213> Artificial Sequence
<220>
   <223> Reverse primer for metA11
<400> 8
   gcactcaagc ttttaatcca gcgttggata catg 34
<210> 9
   <211> 5545
   <212> DNA
   <213> Artificial Sequence
<220>
   <223> pCL_Pcysk-gfp
<210> 10
   <211> 30
   <212> DNA
   <213> Artificial Sequence
<220>
   <223> Forward primer for sucAB
<400> 10
   ggccaagctt gcatcaggcg taacaaagaa 30
<210> 11
   <211> 30
   <212> DNA
   <213> Artificial Sequence
<220>
   <223> Reverse primer for sucAB
<400> 11
   ggccaagctt tgtccatcct tcagtaatcg 30
<210> 12
   <211> 70
   <212> DNA
   <213> Artificial Sequence
<220>
   <223> Forward primer of metB deletion cassette
<210> 13
   <211> 70
   <212> DNA
   <213> Artificial Sequence
<220>
   <223> Reverse primer of metB deletion cassette
<210> 14
   <211> 20
   <212> DNA
   <213> Artificial Sequence
<220>
   <223> Forward primer for deleted metB
<400> 14
   tattcgccgc tccattcagc 20
<210> 15
   <211> 20
   <212> DNA
   <213> Artificial Sequence
<220>
   <223> Reverse primer for deleted metB
<400> 15
   taccccttgt ttgcagcccg 20
<210> 16
   <211> 70
   <212> DNA
   <213> Artificial Sequence
<220>
   <223> Forward primer for thrB deletion cassette
<210> 17
   <211> 70
   <212> DNA
   <213> Artificial Sequence
<220>
   <223> Reverse primer for thrB deletion cassette
<210> 18
   <211> 19
   <212> DNA
   <213> Artificial Sequence
<220>
   <223> Forward primer for deleted thrB
<400> 18
   actcgacgat ctctttgcc 19
<210> 19
   <211> 21
   <212> DNA
   <213> Artificial Sequence
<220>
   <223> Reverse primer for deleted thrB
<400> 19
   acgccgagag gatcttcgca g 21
<210> 20
   <211> 30
   <212> DNA
   <213> Artificial Sequence
<220>
   <223> Forward primer for metA11
<400> 20
   ggccgaattc atgccgattc gtgtgccgga 30
<210> 21
   <211> 30
   <212> DNA
   <213> Artificial Sequence
<220>
   <223> Reverse primer for metA11
<400> 21
   ggccgagttc ttaatccagc gttggattca 30
<210> 22
   <211> 933
   <212> PRT
   <213> Escherichia coli
<220>
   <221> PEPTIDE
   <222> (1)..(933)
   <223> SucA
<210> 23
   <211> 2802
   <212> DNA
   <213> Escherichia coli
<220>
   <221> gene
   <222> (1)..(2802)
   <223> sucA
<210> 24
   <211> 405
   <212> PRT
   <213> Escherichia coli
<220>
   <221> PEPTIDE
   <222> (1)..(405)
   <223> SucB
<210> 25
   <211> 1218
   <212> DNA
   <213> Escherichia coli
<220>
   <221> gene
   <222> (1) .. (1218)
   <223> sucB
<210> 26
   <211> 309
   <212> PRT
   <213> Escherichia coli
<220>
   <221> PEPTIDE
   <222> (1)..(309)
   <223> MetA
<210> 27
   <211> 930
   <212> DNA
   <213> Escherichia coli
<220>
   <221> gene
   <222> (1)..(930)
   <223> metA
<210> 28
   <211> 386
   <212> PRT
   <213> Escherichia coli
<220>
   <221> PEPTIDE
   <222> (1)..(386)
   <223> MetB
<210> 29
   <211> 1161
   <212> DNA
   <213> Escherichia coli
<220>
   <221> gene
   <222> (1)..(1161)
   <223> metB
<400> 29
   atgacgcgta aacaggccac catcgcagtg cgtagcgggt taaatgacga cgaacagtat      60
   ggttgcgttg tcccaccgat ccatctttcc agcacctata actttaccgg atttaatgaa     120
   ccgcgcgcgc atgattactc gcgtcgcggc aacccaacgc gcgatgtggt tcagcgtgcg     180
   ctggcagaac tggaaggtgg tgctggtgca gtacttacta ataccggcat gtccgcgatt     240
   cacctggtaa cgaccgtctt tttgaaacct ggcgatctgc tggttgcgcc gcacgactgc     300
   tacggcggta gctatcgcct gttcgacagt ctggcgaaac gcggttgcta tcgcgtgttg     360
   tttgttgatc aaggcgatga acaggcatta cgggcagcgc tggcagaaaa acccaaactg     420
   gtactggtag aaagcccaag taatccattg ttacgcgtcg tggatattgc gaaaatctgc     480
   catctggcaa gggaagtcgg ggcggtgagc gtggtggata acaccttctt aagcccggca     540
   ttacaaaatc cgctggcatt aggtgccgat ctggtgttgc attcatgcac gaaatatctg     600
   aacggtcact cagacgtagt ggccggcgtg gtgattgcta aagacccgga cgttgtcact     660
   gaactggcct ggtgggcaaa caatattggc gtgacgggcg gcgcgtttga cagctatctg     720
   ctgctacgtg ggttgcgaac gctggtgccg cgtatggagc tggcgcagcg caacgcgcag     780
   gcgattgtga aatacctgca aacccagccg ttggtgaaaa aactgtatca cccgtcgttg     840
   ccggaaaatc aggggcatga aattgccgcg cgccagcaaa aaggctttgg cgcaatgttg     900
   agttttgaac tggatggcga tgagcagacg ctgcgtcgtt tcctgggcgg gctgtcgttg     960
   tttacgctgg cggaatcatt agggggagtg gaaagtttaa tctctcacgc cgcaaccatg     1020
   acacatgcag gcatggcacc agaagcgcgt gctgccgccg ggatctccga gacgctgctg    1080
   cgtatctcca ccggtattga agatggcgaa gatttaattg ccgacctgga aaatggcttc    1140
   cgggctgcaa acaaggggta a                                              1161
<210> 30
   <211> 310
   <212> PRT
   <213> Escherichia coli
<220>
   <221> PEPTIDE
   <222> (1)..(310)
   <223> ThrB
<210> 31
   <211> 933
   <212> DNA
   <213> Escherichia coli
<220>
   <221> gene
   <222> (1)..(933)
   <223> thrB
<400> 31
   atggttaaag tttatgcccc ggcttccagt gccaatatga gcgtcgggtt tgatgtgctc 60
   ggggcggcgg tgacacctgt tgatggtgca ttgctcggag atgtagtcac ggttgaggcg 120
   gcagagacat tcagtctcaa caacctcgga cgctttgccg ataagctgcc gtcagaacca 180
   cgggaaaata tcgtttatca gtgctgggag cgtttttgcc aggaactggg taagcaaatt 240
   ccagtggcga tgaccctgga aaagaatatg ccgatcggtt cgggcttagg ctccagtgcc 300
   tgttcggtgg tcgcggcgct gatggcgatg aatgaacact gcggcaagcc gcttaatgac 360
   actcgtttgc tggctttgat gggcgagctg gaaggccgta tctccggcag cattcattac 420
   gacaacgtgg caccgtgttt tctcggtggt atgcagttga tgatcgaaga aaacgacatc 480
   atcagccagc aagtgccagg gtttgatgag tggctgtggg tgctggcgta tccggggatt 540
   aaagtctcga cggcagaagc cagggctatt ttaccggcgc agtatcgccg ccaggattgc 600
   attgcgcacg ggcgacatct ggcaggcttc attcacgcct gctattcccg tcagcctgag 660
   cttgccgcga agctgatgaa agatgttatc gctgaaccct accgtgaacg gttactgcca 720
   ggcttccggc aggcgcggca ggcggtcgcg gaaatcggcg cggtagcgag cggtatctcc 780
   ggctccggcc cgaccttgtt cgctctgtgt gacaagccgg aaaccgccca gcgcgttgcc 840
   gactggttgg gtaagaacta cctgcaaaat caggaaggtt ttgttcatat ttgccggctg 900
   gatacggcgg gcgcacgagt actggaaaac taa 933
<210> 32
   <211> 309
   <212> PRT
   <213> Artificial Sequence
<220>
   <223> MetA11
<210> 33
   <211> 930
   <212> DNA
   <213> Artificial Sequence
<220>
   <223> metA11
<210> 34
   <211> 15351
   <212> DNA
   <213> Artificial Sequence
<220>
   <223> pCC1BAC_srcO vector

## Claims

1. A microorganism of the genus *Escherichia* having an ability to produce O-succinylhomoserine or succinic acid, wherein an activity of α-ketoglutarate dehydrogenase complex is enhanced compared to an endogenous activity level thereof.

2. The microorganism according to claim 1, wherein the α-ketoglutarate dehydrogenase complex comprises amino acid sequences set forth in SEQ ID NO: 22 and SEQ ID NO: 24.

3. The microorganism according to claim 1, wherein an activity of homoserine O-succinyltransferase is further enhanced compared to an endogenous activity level thereof.

4. The microorganism according to claim 1, wherein activity of at least one of cystathionine gamma-synthase and homoserine kinase, is further weakened compared to corresponding endogenous activity levels, or eliminated.

5. The microorganism according to any one of claims 1 to 4, wherein the microorganism is *Escherichia coli.*

6. A method of producing O-succinylhomoserine or succinic acid, comprising:
culturing a microorganism of the genus *Escherichia* having an ability to produce O-succinylhomoserine or succinic acid in a media, wherein an activity of α-ketoglutarate dehydrogenase complex is enhanced compared to an endogenous activity level thereof; and
recovering O-succinylhomoserine or succinic acid from the culture media or the cultured microorganism.

7. The method according to claim 6, wherein the microorganism is *Escherichia coli.*

## Patentansprüche

1. Mikroorganismus der Gattung *Escherichia,* der die Fähigkeit zur Herstellung von O-Succinylhomoserin oder Bernsteinsäure aufweist, wobei eine Aktivität eines α-Ketoglutarat-Dehydrogenase-Komplexes im Vergleich zu einem endogenen Aktivitätsgrad davon erhöht ist.

2. Mikroorganismus nach Anspruch 1, wobei der α-Ketoglutarat-Dehydrogenase-Komplex Aminosäuresequenzen, die in SEQ ID NO: 22 und SEQ ID NO: 24 dargelegt sind, umfasst.

3. Mikroorganismus nach Anspruch 1, wobei eine Aktivität der Homoserin-O-Succinyltransferase im Vergleich zu einem endogenen Aktivitätsgrad davon weiter erhöht ist.

4. Mikroorganismus nach Anspruch 1, wobei eine Aktivität von Cystathionin-gamma-Synthase und/oder Homoserin-Kinase im Vergleich zu entsprechenden endogenen Aktivitätsgraden weiter geschwächt oder eliminiert ist.

5. Mikroorganismus nach einem der Ansprüche 1 bis 4, wobei der Mikroorganismus *Escherichia coli* ist.

6. Verfahren zum Herstellen von O-Succinylhomoserin oder Bernsteinsäure, Folgendes umfassend:
Kultivieren eines Mikroorganismus der Gattung *Escherichia,* der die Fähigkeit zur Herstellung von O-Succinylhomoserin oder Bernsteinsäure aufweist, wobei eine Aktivität des α-Ketoglutarat-Dehydrogenase-Komplexes im Vergleich zu einem endogenen Aktivitätsgrad davon erhöht ist; und
Gewinnen von O-Succinylhomoserin oder Bernsteinsäure aus den Kulturmedien oder dem kultivierten Mikroorganismus.

7. Verfahren nach Anspruch 6, wobei der Mikroorganismus *Escherichia coli* ist.

## Revendications

1. Microorganisme du genre Escherichia ayant une aptitude à produire de l'O-succinylhomosérine ou de l'acide succinique, dans lequel une activité de complexe α-cétoglutarate déshydrogénase est accrue en comparaison à un niveau d'activité endogène de celui-ci.

2. Microorganisme selon la revendication 1, dans lequel le complexe α-cétoglutarate déshydrogénase comprend des séquences d'acides aminés précisées dans SEQ ID n° : 22 et SEQ ID n° : 24.

3. Microorganisme selon la revendication 1, dans lequel l'activité d'homosérine O-succinyltransférase est davantage accrue en comparaison à un niveau d'activité endogène de celle-ci.

4. Microorganisme selon la revendication 1, dans lequel l'activité d'au moins l'une de la cystathionine gamma-synthase et de l'homosérine kinase est davantage affaiblie en comparaison à des niveaux d'activité endogène correspondants, ou éliminée.

5. Microorganisme selon l'une quelconque des revendications 1 à 4, dans lequel le microorganisme est *Escherichia coli.*

6. Procédé de production d'O-succinylhomosérine ou d'acide succinique, comprenant :
la mise en culture d'un microorganisme du genre *Escherichia* ayant une aptitude à produire de l'O-succinylhomosérine ou de l'acide succinique dans un milieu, dans lequel une activité de complexe α-cétoglutarate déshydrogénase est accrue en comparaison à un niveau d'activité endogène de celui-ci ; et
la récupération d'O-succinylhomosérine ou d'acide succinique à partir du milieu de culture ou du microorganisme mis en culture.

7. Procédé selon la revendication 6, dans lequel le microorganisme est *Escherichia coli.*
